# EUROPÄISCHE PATENTSCHRIFT

(11) **EP 0 910 327 B1**
(45) Veröffentlichungstag und Bekanntmachung des Hinweises auf die Patenterteilung: **19.12.2001**
(21) Anmeldenummer: 97928178.9
(22) Anmeldetag: 12.06.1997
(51) Int. Cl.: A61K 7/00, A61K 7/16

(54) **WÄRMENDE ZAHNPASTE**
HEATING TOOTHPASTE
DENTIFRICE THERMOGENE

(30) Priorität: 21.06.1996 DE 19624870
(43) Veröffentlichungstag der Anmeldung: 28.04.1999
(73) Patentinhaber: Henkel Kommanditgesellschaft auf Aktien, 40589 Düsseldorf-Holthausen (DE)
(72) Erfinder: LASKA, Hans, D-40627 Düsseldorf (DE); PASTURA, Amerigo, D-58453 Witten (DE); WÜLKNITZ, Peter, D-42799 Leichlingen (DE)
(86) Internationale Anmeldenummer: EP9703047
(87) Internationale Veröffentlichungsnummer: WO9749374

(56) Entgegenhaltungen:
- WO-A-97/02802
- DE-C- 359 071
- US-A- 4 647 451

## Beschreibung

Die Erfindung betrifft Mund- und Zahnpflegemittel in Form eines Pulvers, einer Paste oder eines Gels, die unter Verwendung einer Zahnbürste angewendet werden, und die beim Vermischen mit Wasser oder mit dem Speichel des Mundes Hydratationswärme entwickeln.

Es besteht schon seit vielen Jahren der Wunsch, Zahnpasten zu erzeugen, die im Mund eine leichte Temperaturerhöhung bewirken und den Kontakt temperaturempfindlicher Zähne mit der normalerweise kalten Zahnpaste angenehmer machen.

Hierzu hat man gemäß US 3,250,680 wasserfreies Molekularsieb 5A (Zeolith A) in einer Menge von 5 bis 40 Gew.-% in eine Zahnpaste eingesetzt. Solche Zahnreinigungsmittel haben den Nachteil, daß relativ hohe Mengen von mehr als 10 Gew.-% zur Erreichung einer ausreichenden Wärmeentwicklung verwendet werden müssen und man daher bezüglich der Rezepturgestaltung wenig flexibel ist. Auch ist Zeolith A stark alkalisch - was zu unerwünschter Reizung der Mundschleimhaut und des Zahnfleisches führen kann.

Aus DE 23 17 140 C2 sind wäßrige Haut- und Haarbehandlungsmittel bekannt, die beim Hinzufügen von wasserfreiem Calciumchlorid oder Magnesiumsulfat kurz vor der Anwendung auf der Haut durch Hydratation der zugesetzten Salze erwärmt werden. Eine solche Verfahrensweise wäre bei Zahnpasten nicht durchführbar.

Es wurde gefunden, daß sich Zahnpflegemittel mit exotherm hydratisierenden Salzen formulieren lassen, die im Mund eine angenehme Erwärmung durch Hydratation mit der Speichelflüssigkeit hervorrufen.

Gegenstand der Erfindung sind Zahnpflegemittel, die bei Zutritt von Wasser beim Bürsten der Zähne Wärme freisetzen und in einem Träger mit einem Gehalt an mit Wasser mischbaren ein- oder mehrwertigen Alkoholen ein Salz mit negativer Lösungsenthalpie enthalten.

Die erfindungsgemäßen Zahnpflegemittel können als Pasten oder als halbflüssige Lotionen vorliegen, die sich z.B. aus einem flexiblen Behälter durch leichten Druck auf eine Zahnbürste aufbringen lassen. Als flüssiger Träger dient dabei eine wasserfreie Zusammensetzung, die einen Gehalt an Glycolen oder Polyolen mit 2 bis 10 C-Atomen oder Gemische solcher Produkte aufweist und bei 20° C flüssig ist.

Als mit Wasser mischbare Alkohole können z.B. Glycole und Polyole mit 2 - 10 C-Atomen, z.B. 1,2-Propylenglycol, 1,3-Propylenglycol, Dipropylenglycol, Tripropylenglycol, Ethylenglycol, Diethylenglycol, Triethylenglycol, Tetraethylenglycol oder Gemische dieser Verbindungen eingesetzt werden. Als Polyole eignen sich z.B. Glycerin, Erythrit, Trimethylolpropan, Pentaerythrit, Diglycerin, Triglycerin oder Dipentaerythrit. Auch Sorbit und Polyethylenglycole können darin enthalten sein. Bevorzugt sind erfindungsgemäße Zahnpflegemittel, die 35 bis 80 Gew.-% eines Polyols aus der Gruppe Ethylenglycol, Propylenglycol und Glycerin oder Gemische davon enthalten. Einwertige, kurzkettige Alkohole wie z.B. Ethanol können ebenfalls in begrenzten Mengen enthalten sein.

Als Salze mit negativer Lösungsenthalpie sind solche zu verstehen, die sich in Wasser unter Wärmeentwicklung lösen. Dies ist in der Regel dann der Fall, wenn bei der Auflösung Solvate, in Wasser also Hydrate, gebildet werden und die dabei freiwerdende Bildungswärme größer ist als die zur Überwindung der Gitterenergie verbrauchte Wärme. Meist handelt es sich um ganz oder teilweise dehydratisierte Salze, die in Wasser Hydrate bilden. Besonders bevorzugt enthalten die erfindungsgemäßen Zahnpflegemittel 1 bis 20 Gew.-% eines dehydratisierten Salzes aus der Gruppe MgSO₄, MgCl₂ und CaCl₂ oder deren Gemische.

Die Verwendung höherer Anteile an wasserfreiem oder wasserarmem Magnesiumsulfat kann jedoch auch zu unangenehm starker Temperaturentwicklung bis hin zu Verbrennungen der Mundschleimhaut oder des Zahnfleisches führen, wenn nicht durch zusätzliche Maßnahmen dafür Sorge getragen wird, daß die Hydratation weniger heftig erfolgt. Solche Maßnahmen können z. B. darin bestehen, daß man
- ein bereits teilhydratisiertes Salz einsetzt, bei dem nur noch ein Teil der Lösungswärme freigesetzt werden kann
oder
- ein mit hydrophilen, amphiphilen oder hydrophoben Stoffen gecoatetes (beschichtetes) bzw. teilgecoatetes (imprägniertes) Salz verwendet, bei dem der Lösungsvorgang verzögert abläuft, so daß auch die Lösungswärme sich erst nach und nach entwickeln kann.

Selbstverständlich kann man auch gecoatete, imprägnierte und unbeschichtete dehydratisierte Salze im Gemisch einsetzen, damit man sowohl zu Beginn des Zähneputzens eine spontane Erwärmung verspürt als auch während des Putzvorganges durch verzögerte Hydratisierung des beschichteten Salzes eine Aufrechterhaltung der erhöhten Temperatur im Munde wahrnehmen kann.

Als Träger eignet sich z.B. eine pulverförmige Zusammensetzung aus Polierkomponenten und festen, pulverförmigen Polyolen und pulverförmigen Hilfs- und Zusatzmitteln, wie sie für Zahnreinigungspulver üblich sind.

Erfindungsgemäße Mund- und Zahnpflegemittel in Form von Pasten oder Gelen benötigen als Träger eine flüssige, pastöse oder gelartige Formulierung, die bevorzugt unter Verwendung flüssiger mehrwertiger Alkohole, insbesondere von flüssigen Glycolen oder Polyolen - gegebenenfalls im Gemisch mit festen Polyolen oder Polyethylenglycolen - sowie den üblichen Hilfs- und Zusatzmitteln hergestellt werden.

Wenn als dehydratisiertes Salz ein nicht oder nur teilweise beschichtetes Salz oder ein mit wasserlöslichen (hydrophilen oder amphiphilen) Stoffen beschichtetes Salz eingesetzt wird, muß die Trägerformulierung weitgehend wasserfrei sein, damit es nicht zu einer vorzeitigen Wärmeentwicklung kommt.

Man kann aber auch vollständig mit hydrophoben Stoffen, z.B. mit Paraffinen, Fettsäuren, Wachsen, Silikonen oder mit hydrophoben Polymeren beschichtete dehydratisierte Salze einsetzen. In einem solchen Fall kann die Zusammensetzung des Trägers auch Wasser enthalten, ohne daß es bei der Herstellung und Lagerung der Pasten zur Hydratation und Wärmeentwicklung kommt. Die Wärmeentwicklung findet erst statt, wenn beim Bürsten der Zähne die Beschichtung zerstört und die Hydratation des dehydratisierten Salzes eingeleitet wird.

Zusätzlich zu den genannten, obligatorischen Bestandteilen der erfindungsgemäßen Zahnpflegemittel können diese alle bekannten Hilfs- und Zusatzstoffe enthalten, die der Reinigung und Pflege der Zähne und des Zahnfleisches sowie der Gesunderhaltung der Mundhöhle, der Verbesserung des Aussehens und des Geschmacks sowie der Stabilität und Lagerbeständigkeit dienen.

Zur Unterstützung des Putzvorgangs mit der Zahnbürste enthalten Zahnpflegemittel z.B. Putzkörper bzw. Poliermittel. Geeignete Poliermittel sind z.B. Fällungs- und Gelkieselsäuren, Kreide (CaCO₃), Aluminiumhydroxid, Aluminiumoxid und Calciumphosphate. Bevorzugt enhalten erfindungsgemäße Zahnpflegemittel 10 bis 50 Gew.-% einer Polierkomponente aus der Gruppe Kieselsäure, Kreide, Aluminiumhydroxid oder Mischungen davon.

Darüber hinaus können auch anorganische Verdickungsmittel, z.B. Schichtsilikate, Veegum oder verdickende Kieselsäuren eingesetzt werden. Bevorzugt enthalten erfindungsgemäße Zahnpflegemittel 0,5 bis 10 Gew.-% einer verdickenden Kieselsäure, bevorzugt einer Aerogelkieselsäure oder einer pyrogenen Kieselsäure (z.B. vom Typ Aerosil® ).

Weitere Bestandteile, die in den erfindungsgemäßen Zahnpflegemitteln enthalten sein können, sind:
- Tenside, z.B. Aniontenside, Betaintenside, nichtionogene Tenside. Bevorzugt werden schaumstarke anionische Tenside wie z.B. Natriumlaurylsulfat, Natrium-lauroylsarcosinat, Natrium-Acyltaurat, Natrium-monoglycerid-sulfat oder Acylisethionate eingesetzt. Geeignete nichtionische Tenside sind z.B. (oxethylierte) Sorbitanfettsäureester, Fettsäureoxethylate, Fettsäure(mono)glyceride oder deren Oxethylate oder Alkyl-(oligo)-glucoside. Geeignete Betaintenside sind z.B. Fettacylaminopropyldimethyl-acetobetain oder Fettalkyl-dimethyl-acetobetain.
- Aromaöle und Pflanzenextrakte, z.B. Pfefferminzöl, Krauseminzöl, Eukalyptusöl, Anisöl, Fenchelöl, Kümmelöl, Methylacetat, Zimtaldehyd, Vanillin und Thymol sowie Aromastoffe mit Erdbeer-, Himbeer-, Ananas, Apfel- oder Orangengeschmack.
- Lösungsvermittler für Aromen, z.B. PEG-30-Glycerinmonostearat, PEG-60-Glycerintri-hydroxystearinsäureester, oxethyliertes Rizinusöl u.ä.
- Süßungsmittel, z.B. Saccharin-Natrium, Acesulfam-Kalium, Cyclamat, Aspartam oder Süßholzextrakt,
- Antikarieswirkstoffe, wie z.B. Natriumfluorid, Zinnfluorid, Natrium-monofluorophosphat,
- Antizahnsteinwirkstoffe, wie z.B. Natriumpyrophosphat, Natriumtripolyphosphat, 1-Hydroxyethan-1,1-diphosphonsäure oder Azacycloheptan-2,2-diphosphonsäure oder 1-Phosphonopropan-1,2,3-tricarbonsäure oder deren Alkalimetallsalze.
- Organische Verdickungsmittel, z.B. wasserlösliche Polymere wie Carboxymethylcellulose, Hydroxyethylcellulose, Hydroxypropylguar, Carboxymethylstärke, Biopolymere wie Xanthan-Gum, Pflanzengumme wie Gummi arabicum, Traganth, Keraya-Gum, Guar oder synthetische Polymere wie z.B. Polyvinyl-pyrrolidon, Polyvinylalkohol oder hochmolekulares Polyethylenglycol.
- Antimikrobielle Stoffe wie z.B. Chlorhexidin® , Triclosan® , p-Hydroxybenzoesäureester, Natriumsorbat, Natriumbenzoat oder Phenyl-salicylsäureester.
- Pigmente wie z.B. Titandioxid,
- Farbstoffe
- Puffersubstanzen, wie z.B. primäre und sekundäre Alkaliorthophosphate, Natriumcitrat und
- wundheilende und entzündungshemmende Stoffe wie z.B. Allantoin, Azulen, Panthenol oder Harnstoff.

Eine besonders bevorzugte Ausführungsform der erfindungsgemäßen Mund- und Zahnpflegemittel in Form einer Zahncreme enthält
35 - 60 Gew.-% Propylenglycol-1,2 oder Glycerin
30 - 40 Gew.-% einer Poliermittelkomponente
0,5 - 5 Gew.-% einer verdickenden Kieselsäure
2 - 10 Gew.-% MgSO₄ (dehydratisiert)
0,5 - 2 Gew.-% Tenside, z.B. Na-Laurylsulfat
0,2 - 2 Gew.-% Aromaöl
0,05 - 0,5 Gew.-% Süßstoff und
0.2 - 2,0 Gew.-% NaF oder Na₂PO₃F

Die erfindungsgemäßen Zahnpflegemittel können wie übliche Zahnpasten verwendet werden, sie entwickeln aber beim Zähneputzen durch Reaktion (Hydratation) mit dem Wasser der Speichelflüssigkeit Wärme, die bei der angegebenen Dosierung als durchaus angenehm empfunden wird.

Die folgenden Beispiele sollen den Erfindungsgegenstand näher erläutern:

### Beispiele

Es wurden Zahnpastenformulierungen durch Vermischen der Komponenten gemäß Tabelle hergestellt:

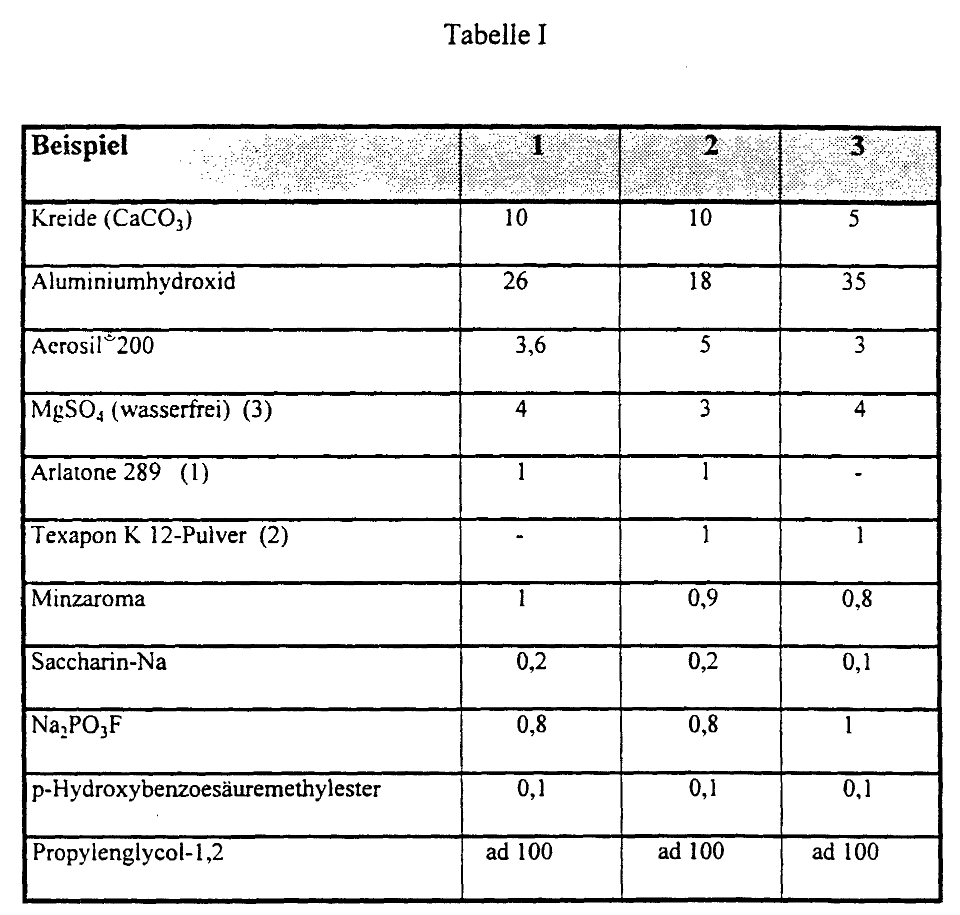

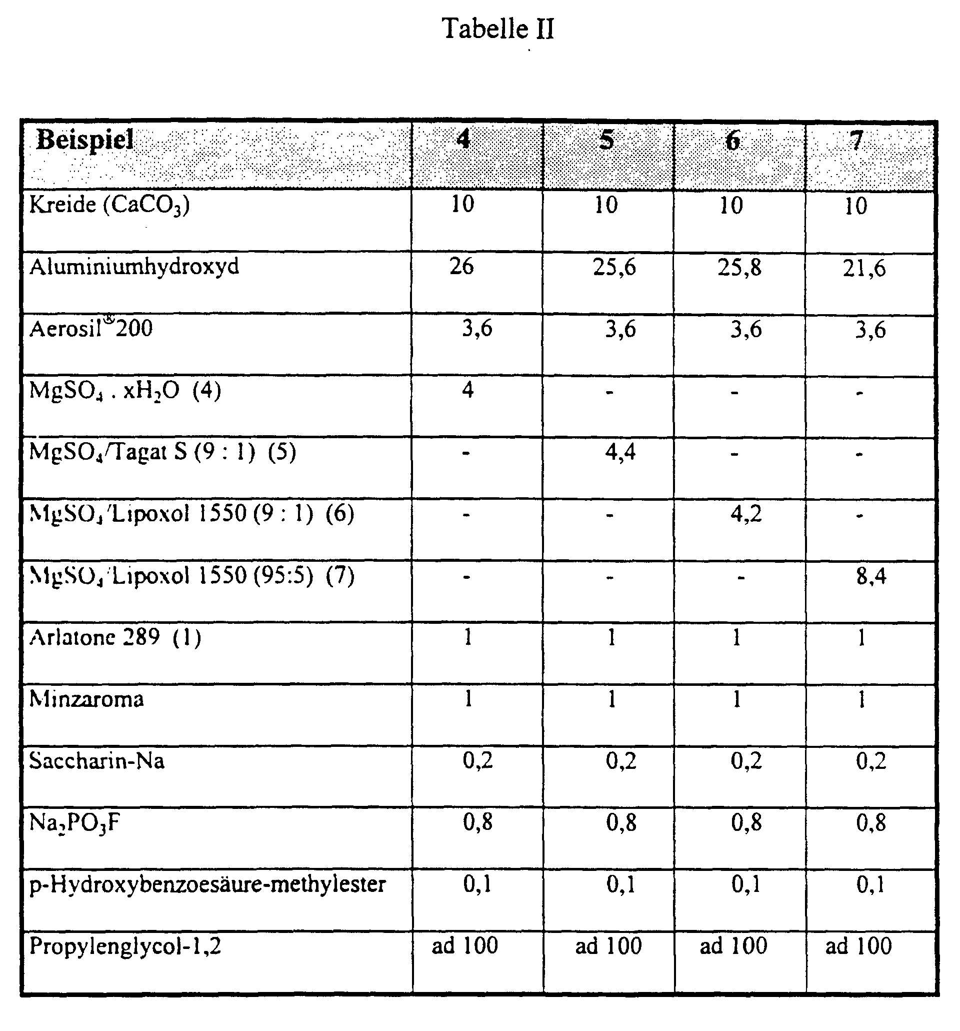

Es wurden folgende Handelsprodukte (Warenzeichen) verwendet:
(1) Arlatone® 289: Fettsäurepolyglycolester, MG: ca. 3315, HLB: ca. 14,4
(2) Texapon® K12-Pulver: Natriumlaurylsulfat
(3) Hersteller: Merck, Darmstadt
(4) Hersteller: Riedel-de-Haen, Trockenverlust (400 - 500°C): 26 - 32 Gew.-%
(5) MgSO₄ (4) gecoatet mit 10 Gew.-% Tagat® S (PEG-30 Glyceryl Stearat, Goldschmidt AG)
(6) MgSO₄ (3) gecoatet mit 10 Gew.-% Lipoxol® 1550 (Polyethylenglycol, MG 1550, Hüls AG)
(7) MgSO₄ (4) gecoatet mit 5 Gew.-% Lipoxol® 1550 (Polyethylenglycol, MG 1550, Hüls AG)

## Patentansprüche

1. Zahnpflegemittel, das bei Zutritt von Wasser beim Bürsten der Zähne Wärme freisetzt und in einem Träger mit einem Gehalt an mit Wasser mischbaren ein- oder mehrwertigen Alkoholen ein dehydratisiertes Salz mit negativer Lösungsenthalpie enthält, **dadurch gekennzeichnet, daß** das Salz Magnesiumsulfat (MgSO₄) ist.

2. Zahnpflegemittel, das bei Zutritt von Wasser beim Bürsten der Zähne Wärme freisetzt und in einem Träger mit einem Gehalt an mit Wasser mischbaren ein- oder mehrwertigen Alkoholen ein dehydratisiertes Salz mit negativer Lösungsenthalpie enthält, **dadurch gekennzeichnet, daß** das Salz ein mit hydrophilen, amphiphilen oder hydrophoben Stoffen beschichtetetes oder imprägniertes Salz ist, bei dem der Lösungsvorgang verzögert abläuft,

3. Zahnpflegemittel nach Anspruch 1 oder 2, **dadurch gekennzeichnet, daß** es 35 - 80 Gew.-% eines Polyols aus der Gruppe Ethylenglycol, Propylenglycol, Glycerin oder deren Mischungen enthält.

4. Zahnpflegemittel nach einem der Ansprüche 1 - 3, **dadurch gekennzeichnet, daß** es 1 - 20 Gew.-% Magnesiumsulfat (MgS04) enthält.

5. Zahnpflegemittel nach einem der Ansprüche 1 bis 4, **dadurch gekennzeichnet, daß** es 10 - 50 Gew.-% einer Polierkomponente aus der Gruppe Aluminiumhydroxid, Kieselsäure, Kreide oder Mischungen davon enthält.

6. Zahnpflegemittel nach einem der Ansprüche 1 bis 5, **dadurch gekennzeichnet, daß** es 0,5 - 10 Gew.-% einer verdickenden Kieselsäure, bevorzugt einer Gelkieselsäure oder pyro-genen Kieselsäure, enthält.

7. Zahnpflegemittel nach einem der Ansprüche 1 - 6, **gekennzeichnet durch** einen Gehalt von
35 - 60 Gew.-% Propylenglycol-1,2 oder Glycerin
30 - 40 Gew.-% einer Poliermittelkomponente
0,5 - 5 Gew.-% einer verdickenden Kieselsäure
2 - 10 Gew.-% MgSO₄ (dehydratisiert)
0,5 - 2 Gew.-% Tenside
0,2 - 2 Gew.-% Aromaöl
0,05 - 0,5 Gew.-% Süßstoff und
0,2 - 2,0 Gew.-% NaF oder Na₂PO₃F

## Claims

1. A dental care preparation which releases heat on contact with water when the teeth are brushed and which contains a dehydrated salt with a negative enthalpy of solution in a carrier containing water-miscible mono- or polyhydric alcohols, **characterized in that** the salt is magnesium sulfate (MgSO₄).

2. A dental care preparation which releases heat on contact with water when the teeth are brushed and which contains a dehydrated salt with a negative enthalpy of solution in a carrier containing water-miscible mono- or polyhydric alcohols, **characterized in that** the salt is a salt which is coated or impregnated with hydrophilic, amphiphilic or hydrophobic substances and which dissolves with delay.

3. A dental care preparation as claimed in claim 1 or 2, **characterized in that** it contains 35 to 80% by weight of a polyol from the group consisting of ethylene glycol, propylene glycol, glycerol or mixtures thereof.

4. A dental care preparation as claimed in any of claims 1 to 3, **characterized in that** it contains 1 to 20% by weight of magnesium sulfate (MgSO₄).

5. A dental care preparation as claimed in any of claims 1 to 4, **characterized in that** it contains 10 to 50% by weight of a polishing component from the group consisting of aluminium hydroxide, silica, chalk or mixtures thereof.

6. A dental care preparation as claimed in any of claims 1 to 5, **characterized in that** it contains 0.5 to 10% by weight of a thickening silica, preferably a gel silica or pyrogenic silica.

7. A dental care preparation as claimed in any of claims 1 to 6, **characterized by** a content of
35 to 60% by weight of 1,2-propylene glycol or glycerol,
30 to 40% by weight of a polishing component,
0.5 to 5% by weight of a thickening silica,
2 to 10% by weight of MgSO₄ (dehydrated),
0.5 to 2% by weight of surfactants,
0.2 to 2% by weight of flavouring oil,
0.05 to 0.5% by weight of sweetener and
0.2 to 2.0% by weight of NaF or Na₂PO₃F.

## Revendications

1. Dentifrice qui par entrée d'eau lors du brossage des dents, libère de la chaleur, et qui contient dans un support avec une teneur en alcools mono- ou polyfonctionnels miscibles à l'eau, un sel déshydraté ayant une enthalpie négative de mise en solution,
**caractérisé en ce que**
le sel est du sulfate de magnésium (MgSO₄).

2. Dentifrice qui par entrée lors du brossage des dents libère de la chaleur et qui contient dans un support, ayant une teneur en alcools mono- ou polyfonctionnels miscibles à l'eau, un sel déshydraté ayant une enthalpie négative de mise en solution,
**caractérisé en ce que**
le sel est un sel revêtu avec des substances hydrophiles, amphiphiles ou hydrophobes ou imprégné pour lequel le processus de mise en solution se déroule d'une manière différée.

3. Dentifrice selon l'une quelconque des revendications 1 ou 2,
**caractérisé en ce qu'**
il contient de 35 à 80 % en poids d'un polyol choisi dans le groupe de l'éthylèneglycol, du propylèneglycol, du glycérol ou de leurs mélanges.

4. Dentifrice selon l'une quelconque des revendications 1 à 3,
**caractérisé en ce qu'**
il contient de 1 à 20 % en poids de sulfate de magnésium (MgSO₄).

5. Dentifrice selon l'une quelconque des revendications 1 à 4,
**caractérisé en ce qu'**
il contient de 10 à 50 % en poids d'un composant de polissage choisi dans le groupe de l'hydroxyde d'aluminium, l'acide silicique, la craie et les mélanges de ceux-ci.

6. Dentifrice selon l'une quelconque des revendications 1 à 5,
**caractérisé en ce qu'**
il contient de 0,5 à 10 % en poids d'un acide silicique destiné à épaissir, de préférence un acide silicique en gel ou de l'acide silicique pyrogénique.

7. Dentifrice selon l'une quelconque des revendications 1 à 6,
**caractérisé par**
une teneur de
35 - 60 % en poids de propylèneglycol-1,2 ou de glycérol,
30 - 40 % en poids d'un composant agent de polissage,
0,5 - 5 % en poids d'un acide silicique destiné à épaissir,
2 - 10 % en poids de MgSO₄ (déshydraté),
0,5 - 2 % en poids de détergent,
0,2 - 2 % en poids d'huile aromatique,
0,05 - 0,5 % en poids d'édulcorant et
0,2 - 2,0 % en poids de NaF ou Na₂PO₃F.
